(19) Europäisches Patentamt / European Patent Office / Office européen des brevets

(11) **EP 3 766 498 A1**

(12) **EUROPEAN PATENT APPLICATION**

(43) Date of publication:
**20.01.2021 Bulletin 2021/03**

(51) Int Cl.:
**A61K 31/575** (2006.01) **A61K 9/14** (2006.01)
**A61K 35/413** (2015.01) **A61P 9/00** (2006.01)

(21) Application number: **20185185.4**

(22) Date of filing: **10.07.2020**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(30) Priority: **17.07.2019 CN 201910646710**<br><br>(71) Applicant: **Shanghai Kaibao Pharmaceutical Co., Ltd.**<br>**Shanghai 201401 (CN)** | (72) Inventors:<br>• **GUO, Qiuping**<br>  **Shanghai, 201401 (CN)**<br>• **MU, Jingwei**<br>  **Shanghai, 201401 (CN)**<br>• **CHEN, Dixin**<br>  **Shanghai, 201401 (CN)**<br>• **LI, Ruipeng**<br>  **Shanghai, 201401 (CN)**<br>• **ZHANG, Xiaoli**<br>  **Shanghai, 201401 (CN)**<br><br>(74) Representative: **Cabinet Chaillot**<br>**16/20, avenue de l'Agent Sarre**<br>**B.P. 74**<br>**92703 Colombes Cedex (FR)** |

(54) **USE OF BIO-TRANSFORMED BEAR BILE POWDER IN PREPARATION OF ANTI-INFLAMMATORY DRUGS**

(57) A use of bio-transformed bear bile powder in preparation of anti-inflammatory drugs is disclosed. The present invention carries out a comparative research on the anti-inflammatory drug activity of bio-transformed bear bile powder and natural bear bile powder. A research is made on the effect of the bio-transformed bear bile powder and natural bear bile powder on carrageenan-induced rat inflammation models, and a research is made on the effect of the bio-transformed bear bile powder on LPS-induced lung inflammation in mice. Experimental results show that the bio-transformed bear bile powder administration group obviously reduces plantar swelling of carrageenan-induced inflammation model in rats, and the effect thereof is not lower than that of the natural bear bile powder. Both the bio-transformed bear bile powder and the natural bear bile powder can significantly reduce levels of the inflammatory factors IL-6 and TNF-$\alpha$ in the lung and the serum. It is therefore that the bio-transformed bear bile powder can be used to prepare the anti-inflammatory drugs.

**Description**

**BACKGROUND OF THE INVENTION**

**1. Field of the Invention**

The invention relates to the field of pharmaceuticals, and more particularly, to a use of bio-transformed bear bile powder in preparation of anti-inflammatory drugs.

**2. Description of the Related Art**

Bear bile is obtained from the dried gallbladder bile of black bear or brown bear belonging to the family Ursidae. It could clear heat, clear away liver fire, and improve eyesight. The bear bile is mainly composed of tauro-ursodeoxycholic acid (TUDCA). The bear bile is regarded as one of the four valuable medicines made from animals. The bear bile has been used in Traditional Chinese Medicine for about two thousand years. Many prescription drugs contain the bear bile. The annual output of bear bile in China is about 30 tons, however, it still could not meet the demand. At present, due to a great imbalance between supply and demand of high-end bear bile and a continuous increase in the demand for the bear bile utilized for low-end raw materials, the price of bear bile rises. The market price for the bear bile has exceeded 5000 RMB/kg. Worse still, since animal conversation groups, both home and abroad, have long resisted the practice of extracting bear bile from bears alive, and particularly, the "Gui Zhen Tang (a pharmaceuticals company)" incident occurred recently, extraction of bear bile from the live bears has been a focus of the international community. Consequently, the production of bear bile and its related industries are confronted with unprecedented challenges. Therefore, speeding up R&D of artificial bear bile plays a key role in promoting the sustainable development of the bear bile industry.

An artificial bear bile powder and a preparation method thereof are provided in Chinese Patent Application CN 201410588581.5 (Patent No. CN 104382941B), entitled "Artificial bear bile and preparation method thereof". In this method, biocatalysis and biotransformation of livestock bile are simulated *in vitro* without adding other chemical compositions; the livestock bile is made into artificial bear bile by a certain process. However, there has not been any research on the use of the artificial bear bile powder in preparation of drugs.

**SUMMARY OF THE INVENTION**

In order to solve the problems in the prior art, the present invention provides a use of bio-transformed bear bile powder in preparation of anti-inflammatory drugs.

For the above-mentioned purposes, the present invention adopts the following technical solution:
It is known from the Chinese Patent Application CN 104382941B that the bio-transformed bear bile powder is prepared by the following method:

a) livestock bile powder is dissolved with a buffer solution of pH 7-9 and centrifuged at 11000 g for 10 minutes; the solution with the livestock bile powder is filtered several times to obtain filter residue and filtrate; the filter residue is then lyophilized and dried for store; the livestock bile powder is one or more selected from the group consisting of chick bile powder, duck bile powder, goose bile powder, cow bile powder, rabbit bile powder, dog bile powder, and sheep bile powder, wherein the main composition and mass percentage are as follows: 40%-65% of total bile acids, 4%-9% amino acids, 0.5%-1% microelements, wherein the total bile acids contain TCDCA (Taurochenodeoxycholic acid), and the content (wt%) of TCDCA is in a range of 40-50%, based on the total weight of the livestock bile powder;

b) the filtrate is added to a reactor filled with 7$\alpha$-hydroxysteroid dehydrogenase, 7$\beta$-hydroxysteroid dehydrogenase and Coenzyme I or Coenzyme II for reaction; the 7$\alpha$-hydroxysteroid dehydrogenase and the 7$\beta$-hydroxysteroid dehydrogenase exist in the reactor in an immobilized manner;

c) after the reaction is completed, the reaction solution is lyophilized and dried for 48 hours and mixed with the filter residue to obtain the bio-transformed bear bile powder.

According to the first aspect, the present invention provides a use of bio-transformed bear bile powder in preparation of anti-inflammatory drugs.

In another preferred embodiment, the anti-inflammatory drugs comprise bio-transformed bear bile powder and pharmaceutically acceptable excipients.

In another preferred embodiment, the dosage form of the anti-inflammatory drugs comprises oral dosage forms or non-oral dosage forms.

In another preferred embodiment, the oral dosage forms comprise tablets, powders, granules, capsules, emulsions, syrups, or sprays.

In another preferred embodiment, the non-oral dosage forms comprise injections, external preparations.

In another preferred embodiment, the external preparations are eye drops.

In another preferred embodiment, the bio-transformed bear bile powder is artificial bear bile powder which is prepared from poultry bile as raw material through biological fermentation.

In another preferred embodiment, the poultry bile is extracted from one or more selected from the group consisting of chick bile powder, duck bile powder, goose bile powder, cow bile powder, rabbit bile powder, dog bile powder, and sheep bile powder, preferably, the chick bile powder.

According to the second aspect, the present invention provides a use of bio-transformed bear bile powder in preparation of anti-pneumonia drugs.

In another preferred embodiment, the anti-pneumonia drugs comprise bio-transformed bear bile powder and pharmaceutically acceptable excipients.

In another preferred embodiment, the dosage form of the anti-pneumonia drugs comprises oral dosage forms or non-oral dosage forms.

In another preferred embodiment, the oral dosage forms comprise tablets, powders, granules, capsules, emulsions, syrups, or sprays.

In another preferred embodiment, the non-oral dosage forms comprise injections, external preparations.

In another preferred embodiment, the external preparations are eye drops.

In another preferred embodiment, the artificial bear bile powder is artificial bear bile powder prepared from poultry bile as raw material through biological fermentation.

In another preferred embodiment, the poultry bile is extracted from one or more selected from the group consisting of chick bile powder, duck bile powder, goose bile powder, cow bile powder, rabbit bile powder, dog bile powder, and sheep bile powder, preferably, the chick bile powder.

By adopting the above-mentioned technical solutions, the present invention has the following advantages over the prior art:

the present invention carries out a comparative research on the anti-inflammatory drug activity of bio-transformed bear bile powder and natural bear bile powder. In an animal experiment, a research is made on the effects of the bio-transformed bear bile powder on models of inflammation in rats, and a research is made on the effects of the bio-transformed bear bile powder on inflammation in LPS-induced mice. Experimental results show that the bio-transformed bear bile powder has as much anti-inflammatory effects as the natural bear bile powder does. Therefore, the bio-transformed bear bile powder enjoys a great prospect in preparation of anti-inflammatory drugs.

It should be understood that within the scope of the present invention, the above technical features of the present invention and the technical features specifically described hereinafter (e.g., in examples) can be combined with each other, thereby forming a new or preferred technical solution. Due to space limitations, details will not be repeated herein.

## BRIEF DESCRIPTION OF THE DRAWINGS

The accompanying drawings, together with the specification, illustrate exemplary embodiments of the present disclosure, and, together with the description, serve to explain the principles of the present invention.

Figure 1 is a graph showing the effect of bio-transformed bear bile powder on the plantar swelling of carrageenan-induced inflammation model in rats in Example 1;

Figure 2 is a graph showing the effect of natural bear bile powder on the plantar swelling of carrageenan-induced inflammation model in rats in Example 1;

Figure 3 is a graph showing the effect of administration of bear bile powder for 7 consecutive days on the inflammatory factors IL-6 in LPS-induced lung inflammation serum in mice in Example 2;

Figure 4 is a graph showing the effect of administration of bear bile powder for 7 consecutive days on the inflammatory factors IL-6 in LPS-induced lung inflammation alveolar lavage fluid in mice in Example 2;

Figure 5 is a graph showing the effect of administration of bear bile powder for 7 consecutive days on the inflammatory factors TNF-$\alpha$ in LPS-induced lung inflammation serum in mice in Example 2;

Figure 6 is a graph showing the effect of administration of bear bile powder for 7 consecutive days on the inflammatory factors TNF-$\alpha$ in LPS-induced lung inflammation alveolar lavage fluid in mice in Example 2;

Figure 7 is a microscope image of lung tissue subjected to HE (hematoxylin-eosin) staining under a 20x objective lens in Example 2, wherein A is a control group, B is a model group, I is a natural bear bile powder 1000 mg/kg group, and J is a bio-transformed bear bile powder 1000 mg/ kg group; and

Figure 8 is a statistical graph showing the lung injury score in Example 2, wherein A is a control group, B is a model group, I is a natural bear bile powder 1000 mg/kg group, and J is a bio-transformed bear bile powder 1000 mg/ kg group.

**DETAILED DESCRIPTION**

Bio-transformed bear bile powder is made from chick bile powder by a method comprising the steps of:

step 1, preparation of chick bile powder: the chick bile is purchased from a slaughterhouse, and the number and weight of the chick bile is determined; the chick bile is topically sterilized with 75% by weight of alcohol, and the chick bile is cut open for taking out bile; the bile taken out from the chick bile is lyophilized and dried for 48 hours to obtain chick bile powder; and the obtained chick bile powder is placed in a dryer for future use;

step 2, treatment of the chick bile powder: the chick bile powder is dissolved with a buffer solution of Ph 7-9 to prepare 1L solution, and the solution of the chick bile powder is fully dissolved using ultrasound; the solution of the chick bile powder is centrifuged at 11000 g for 10 minutes and filtered several times; the supernatant (i.e., the filtrate) is stored at 4 °C as the treatment liquid of the livestock bile powder for future use; the filter residue is then lyophilized and dried, and is stored for future use;

wherein, the buffer solution is one selected from phosphate buffer solution (PBS), Tris-HCl buffer (Tris(hydroxymethyl)aminomethane), or glycine-sodium hydroxide buffer Gly-NaOH;

step 3, preparation of immobilized enzyme: an appropriate amount of acetic acid is added to a 1% by weight of chitosan (water-soluble), the resulting solution is magnetically stirred for 30 minutes to prepare a chitosan acetic acid solution, the chitosan acetic acid solution is injected into 100 mL sodium tripolyphosphate solution of 3% by weight by using a high-pressure syringe pump and is cured for 1 to 2 hours, to obtain white chitosan balls having a uniform particle size and a regular shape; the solution is filtered and washed with deionized water multiple times. The prepared chitosan balls are placed in a beaker containing 0.125% by weight of glutaraldehyde aqueous solution and plated in a shaker at 170 g, at 37 °C for activation for 2 to 4 hours, so as to obtain activated chitosan microspheres.

10 g chitosan microspheres which are activated, are weighed and placed in a small chromatography column, an appropriate amount of 7α-hydroxysteroid dehydrogenase and 7β-hydroxysteroid dehydrogenase are added; the chitosan microspheres and the added 7α-hydroxysteroid dehydrogenase and 7β-hydroxysteroid dehydrogenase are mixed for 4 to 6 hours by using vertical mixing method. After the enzyme column is immobilized, the enzyme solution is released. The immobilized enzyme column is rinsed with PBS for 3 to 5 times. The immobilization rate of 7α-hydroxysteroid dehydrogenase and 7β-hydroxysteroid dehydrogenase is greater than 90%.

Wherein, 7α-hydroxysteroid dehydrogenase (7α-HSDH) and 7β-hydroxysteroid dehydrogenase (7β-HSDH) are derived from microorganisms, such as *Clostridium sardiniense* (DSM599/ATCC27555), *Bacteroides fragilis* (ATCC25825) or *Collinsella aerofaciens* (ATCC25986).

Step 4, biocatalysis and biotransformation: the prepared livestock bile powder treatment liquid is added to the immobilized enzyme column as reaction substrate solution, coenzyme I or coenzyme II are added, so that a ratio (molar ratio) of the coenzyme I (or the coenzyme II) to TCDCA is in a range of 1:2 to 1:4, and the resulting solution is reacted at a temperature of 20 °C to 30 °C for 2 to 6 hours.

Step 5, treatment of the reaction solution: the reaction solution reacted in the step 4 is lyophilized and dried for 48 hours to obtain a solid reaction product, and finally, the filter residue obtained in the step 2 is uniformly mixed with the above-mentioned solid reaction product to obtain the bio-transformed bear bile powder.

The above-mentioned bio-transformed bear bile powder can be used in preparation of anti-inflammatory drugs.

In another preferred embodiment, the anti-inflammatory drugs comprise bio-transformed bear bile powder and pharmaceutically acceptable excipients.

In another preferred embodiment, the dosage form of the anti-inflammatory drugs comprises oral dosage forms or non-oral dosage forms.

In another preferred embodiment, the oral dosage forms comprise tablets, powders, granules, capsules, emulsions, syrups, or sprays.

In another preferred embodiment, the non-oral dosage forms comprise injections, external preparations.

In another preferred embodiment, the external preparations are eye drops.

In another preferred embodiment, the bio-transformed bear bile powder is artificial bear bile powder which is prepared from poultry bile as raw material through biological fermentation.

In another preferred embodiment, the poultry bile is extracted from one or more selected from the group consisting of chick bile powder, duck bile powder, goose bile powder, cow bile powder, rabbit bile powder, dog bile powder, and sheep bile powder, preferably, the chick bile powder.

The above-mentioned bio-transformed bear bile powder can be used in preparation of anti-pneumonia drugs.

In another preferred embodiment, the anti-pneumonia drugs comprise bio-transformed bear bile powder and pharmaceutically acceptable excipients.

In another preferred embodiment, the dosage form of the anti-pneumonia drugs comprises oral dosage forms or non-oral dosage forms.

In another preferred embodiment, the oral dosage forms comprise tablets, powders, granules, capsules, emulsions, syrups, or sprays.

In another preferred embodiment, the non-oral dosage forms comprise injections, external preparations.

In another preferred embodiment, the external preparations are eye drops.

In another preferred embodiment, the bio-transformed bear bile powder is artificial bear bile powder which is prepared from poultry bile as raw material through biological fermentation.

In another preferred embodiment, the poultry bile is extracted from one or more selected from the group consisting of chick bile powder, duck bile powder, goose bile powder, cow bile powder, rabbit bile powder, dog bile powder, and sheep bile powder, preferably, the chick bile powder.

The present invention will be described hereinafter with reference to particular embodiments, but the invention is not limited thereto.

**Example 1**

Research of the Effect of Bio-transformed Bear Bile Powder and Natural Bear Bile Powder on Carrageenan-Induced Inflammation Model in Rats

Experiment is carried out as follows:

Animal germline: specific pathogen-free (SPF) male SD rats, aged 6-week-old, weighing between 132 g and 199 g when received, and weighing between 152 g and 225 g when drugs are administered to rats, were purchased from Hunan Slake Jingda laboratory Animal Co., Ltd.

The laboratory animal production license: SCXK (Xiang) 2016-0002 (valid until September 29, 2021), issued by China Hunan Provincial Science & Technology Department. The laboratory animal quality certificate No.: 43004700060779.

Method for grouping animals: after the quarantine was over, rats that have passed the quarantine inspection were selected for experiments. Groups were randomly assigned on the basis of body weight and divided into 9 groups with 10 rats per group. The 9 groups were as follows: a negative control group, a model group, a group of rats treated with low-dose bio-transformed bear bile powder, a group of rats treated with middle-dose bio-transformed bear bile powder, a group of rats treated with high-dose bio-transformed bear bile powder, a group of rats treated with low-dose natural bear bile powder, a group of rats treated with middle-dose natural bear bile powder, a group of rats treated with high-dose natural bear bile powder, and a positive control group.

Negative control and solvent: the negative control was 05% by weight of sodium carboxymethyl cellulose solution (CMC-Na) (analytically purity). For the solution, the specification was 500 g/bottle, batch number was 20150806, and the manufacturer was Tianjin Damao Chemical Regent Factory. Temperature and humidity were not particularly limited when preparing the regent, CMC-Na was weighed and dissolved with ultrapure water, so as to prepare a CMC-Na solution at a concentration of 0.5% by weight. The solution was stored at room temperature without ant limitations on humidity. The shelf life is 7 days. The solvent: both the test substance and the positive control drug were 0.5% by weight of sodium carboxymethyl cellulose solution (CMC-Na) (analytically purity).

Dose and group design for the experiment: a total of 9 groups are contemplated, that is, negative control group (blank control group), a model group, a group of rats treated with low-dose bio-transformed bear bile powder, a group of rats treated with middle-dose bio-transformed bear bile powder, a group of rats treated with high-dose bio-transformed bear bile powder, a group of rats treated with low-dose natural bear bile powder, a group of rats treated with middle-dose natural bear bile powder, a group of rats treated with high-dose natural bear bile powder, and a positive control group (a group of rats treated with indomethacin). Rats were divided into 9 groups with 10 rats per group, male, and details were shown in Table 1 below.

Table 1

| Group No. | Number of animal | Groups | Dose (g/kg) | Concentration of test substance (g/mL) | Administration volume (mL/kg) |
|---|---|---|---|---|---|
| A | 10 | blank control group | | | 10 |
| B | 10 | model group | | | 10 |
| C | 10 | a group of rats treated with low-dose bio-transformed bear bile powder | 0.046 | 0.0046 | 10 |
| D | 10 | a group of rats treated with middle-dose bio-transformed bear bile powder | 0.093 | 0.0093 | 10 |

(continued)

| Group No. | Number of animal | Groups | Dose (g/kg) | Concentration of test substance (g/mL) | Administration volume (mL/kg) |
|---|---|---|---|---|---|
| E | 10 | a group of rats treated with high-dose bio-transformed bear bile powder | 0.185 | 0.0185 | 10 |
| F | 10 | a group of rats treated with low-dose natural bear bile powder | 0.045 | 0.0045 | 10 |
| G | 10 | a group of rats treated with middle-dose natural bear bile powder | 0.09 | 0.009 | 10 |
| H | 10 | a group of rats treated with high-dose natural bear bile powder | 0.18 | 0.018 | 10 |
| I | 10 | positive control group | 0.007 | 0.0007 | 10 |

Each group in Table 1 was administrated at 10 mL/kg, and the negative control group and the model group were assigned with 0.5% by weight of CMC-Na solution.

Administration route, administration frequency, and administration period of the test substance and the control: each of them was administered intragastrically, the administration volume was 10 mL/kg, and each group was administered once.

Experimental method: SD male rats that had passed the quarantine inspection were selected for experiments. Groups were divided into 9 groups with 10 male rats per group. The 9 groups were as follows: a negative control group, a model group, a group of rats treated with low-dose bio-transformed bear bile powder, a group of rats treated with middle-dose bio-transformed bear bile powder, a group of rats treated with high-dose bio-transformed bear bile powder, a group of rats treated with low-dose natural bear bile powder, a group of rats treated with middle-dose natural bear bile powder, a group of rats treated with high-dose natural bear bile powder, and a positive control indomethacin group. Before molding, each animal was scored at the ankle joint of the right hind foot, and the volume of the right hind foot was measured with a plantar volume measuring instrument, and it was measured continuously 2 times, wherein the average value obtained from the two measurements was taken as a base volume.

Except for the blank control group, the animals in the remaining groups were injected with 1% by weight of carrageenan suspension at the right hind foot metatarsal palmar fascia, the volume for each rat was 0.1 mL; animals in the blank control group were injected with an equal volume of normal saline; corresponding drugs were administered intragastrically immediately after an injection of carrageenan. The plantar volume of the right hind foot of each group of animals was measured at 1h, 2h, 3h, 4h, 5h, and 6h after injection, and was measured twice consecutively, and the average value obtained from the two measurements was taken as the plantar volume after modeling. The plantar swelling and the plantar swelling inhibition rate were calculated according to the plantar volume measured at different time points after inflammation was induced.

$$\text{Swelling inhibition rate (\%)} = (\text{average swelling degree of model group} - \text{average swelling degree of drug administration group})/\text{average swelling degree of model group} \times 100\%$$

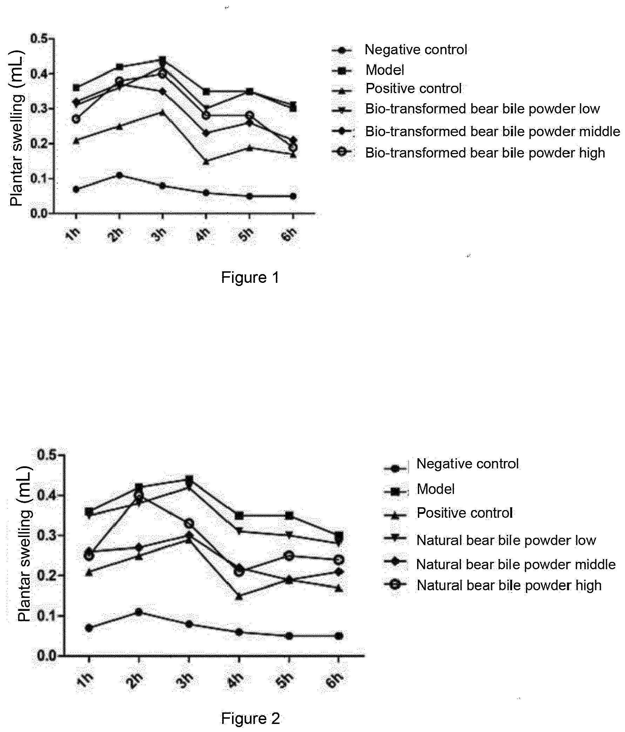

Analysis method for experimental statistics: all data were input into EXCEL for statistical analysis. In different groups, the mean $\pm$ standard deviation of plantar swelling are calculated. SPSS software was used to test the normal distribution of data between each group, and single factor variance is used to analyze and compare the differences between groups. Before the comparison, the data were subjected to homogeneity test of variance. When the variances between the groups were equal, LSD, Bonferroni and SNK tests were used for statistical analysis; when the variances between the groups are unequal, Dunnett's T3 test, Dunnett's C test were used for statistical analysis. Test results were shown in Tables 2- 3, and Figures 1-2.

In table 2, compared with the negative control group, # means $p<0.05$; compared with the model group, * means $p<=0.05$.

Table 2

| Groups | Dose (g/kg) | Base foot (mL) | Plantar swelling (mL) | | | | | |
|---|---|---|---|---|---|---|---|---|
| | | | volume 1 h | 2 h | 3 h | 4 h | 5 h | 6 h |
| negative control group | | 2.12±0.27 | 0.07±0.06 | 0.11±0.09 | 0.08±0.06 | 0.06±0.03 | 0.05±0.06 | 0.02±0.04 |
| model group | - | 2.04±0.21 | 0.36±0.15# | 0.42±0.14# | 0.44±.21# | 0.35±0.15# | 0.35±0.12# | 0.30±0.13* |
| positive control group | 0.007 | 2.22±0.26 | 0.21±0.14* | 0.25±0.16* | 0.29±0.15 | 0.15±0.12* | 0.19±0.09* | 0.17±0.10* |
| a group ofrats treated with low-dose bio-transformed bear bile powder | 0.046 | 2.05±0.10 | 0.31±0.20NS | 0.36±0.15 NS | 0.42±0.10 NS | 0.30±0.09 NS | 035±0.15 NS | 0.31±0.16NS |
| a group of rats treated with middle-dose bio-transformed bear bile powder | 0.093 | 2.04±0.12 | 0.32±0.22 NS | 0.37±0.14 NS | 0.35±0.15 NS | 0.23±0.09* NS | 0.26±0.14 NS | 0.21±0.10NS |
| a group of rats treated with high-dose bio-transformed bear bile powder | 0.185 | 2.01±0.01 | 0.27±0.14 NS | 0.38±0.11 NS | 0.40±0.13 NS | 0.28±0.14 NS | 0.28±0.10 NS | 0.19±0.10*NS |
| a group of rats treated with low-dose natural bear bile powder | 0.045 | 2.04±0.01 | 0.35±0.16 | 0.38±0.11 | 0.42±0.11 | 0.31±0.18 | 0.30±0.16 | 0.28±0.13 |
| a group of rats treated with middle-dose natural bear bile powder | 0.09 | 2.06±0.01 | 0.26±0.14 | 0.27±0.12* | 0.30±0.14 | 0.22±0.10 | 0.19±009* | 0.21±0.13 |
| a group of rats treated with high-dose natural bear bile powder | 0.18 | 2.09±0.01 | 0.25±0.19 | 0.40±0.21 | 0.33±0.12 | 0.21±0.14* | 0.25±0.18 | 0.24±0.18 |

Table 3

| Groups | Dose(g crude drug/kg) | Swelling inhibition rate (%) | | | | | |
|---|---|---|---|---|---|---|---|
| | | 1 h | 2 h | 3 h | 4 h | 5 h | 6 h |
| positive control group | 0.007 | 40.03 | 39.71 | 33.75 | 56.51 | 46.51 | 44.74 |
| a group of rats treated with low-dose bio-transformed bear bile powder | 0.046 | 13.48 | 14.15 | 5.66 | 12.88 | 1.42 | -4.51 |
| a group of rats treated with middle-dose bio-transformed bear bile powder | 0.093 | 9.13 | 12.01 | 20.39 | 33.19 | 27.17 | 28.88 |
| a group of rats treated with high-dose bio-transformed bear bile powder | 0.185 | 24.44 | 10.23 | 9.06 | 20.17 | 20.77 | 36.73 |
| a group of rats treated with low-dose natural bear bile powder | 0.045 | 1.54 | 10.82 | 4.08 | 10.44 | 14.08 | 5.68 |
| a group of rats treated with middle-dose natural bear bile powder | 0.09 | 26.54 | 35.43 | 32.05 | 35.91 | 45.95 | 29.88 |
| a group of rats treated with high-dose natural bear bile powder | 0.18 | 30.20 | 4.99 | 25.14 | 40.63 | 29.59 | 21.04 |

Experimental results showed that the plantar swelling of the animals in the model groups increased significantly (p<0.01) compared with that of the negative control group, indicating that carrageenan has successfully caused inflammation, and successful modeling was achieved. When compared with the model group, the plantar swelling of the animals in the bio-transformed bear bile powder 0.093g/kg, 0.185g/kg, and in the natural bear bile powder 0.09g/kg, 0.18g/kg decreased (p<0.05 or p<0.01), and the effect of the bio-transformed bear bile powder on the relief of the plantar swelling was more obvious. This indicated that the bio-transformed bear bile powder group had better anti-inflammatory effects on carrageenan-induced inflammation model animals than the natural bear bile powder did.

It can be seen that the pharmacodynamic activity of bio-transformed bear bile powder in inflammation is comparable to that of natural bear bile powder, indicating that bio-transformed bear bile powder can be used to prepare anti-inflammatory drugs and has better pharmacodynamic activity.

**Example 2**

Research of the Effect of Bio-transformed Bear Bile Powder and Natural Bear Bile Powder on LPS-Induced Lung Inflammation in Rats

Experiment is carried out as follows:

Animal germline: 45 male C57BL/6 mice, purchased from Shanghai Lingchang Biological Technology Co., Ltd. The laboratory animal production license: SCXK(Hu) 2013-0018.

Dose and group design for the experiment: a total of 4 groups are contemplated, that is, a blank control group, LPS model control group, a group of mice treated with a dose of 1000 mg/kg bio-transformed bear bile powder, and a group of mice treated with a dose of 1000 mg/kg natural bear bile powder.

Experimental method: mice in the LPS model control group and mice in other administration groups were exposed to nebulized LPS (2.5 mg/ml) twice daily (each exposure lasted for 30 minutes, and the interval between the two exposures was 2 hours) for 7 consecutive days. Once the second nebulization was completed each day, mice in each group were given different reagents. Blood was collected from orbit and placed in a centrifuge tube at five hours after the end of the second nebulization on days 1, 4, and 7 of the experiment. The collected blood was placed at room temperature for 2 hours and centrifuged at 2000 g for 20 minutes to separate serum The blood with the serum separated was cryopreserved at -80°C for future use, and the levels of IL-6 and TNF-$\alpha$ in the serum were detected. After blood was collected on day 7 of the experiment, the chest cavity of the mouse was opened. The left lung lobe of the mouse was ligated, intubated from the trachea, and rinsed with 0.5 ml PBS 3 times, and the recovered solution was taken out and placed in a centrifuge tube. The recovered solution was centrifuged at 4°C, at 1500 rpm for 10 minutes to seperate supernatant, and it was stored at -80°C in a freezer. The levels of IL-6 and TNF-$\alpha$ in bronchocalveolar lavage fluid were detected. Lung tissues, which had been ligated, were taken and placed into formalin solution for immobilization. The lung samples of 5 mice were randomly selected from each group for sectioning and HE staining for histopathological examination.

**EP 3 766 498 A1**

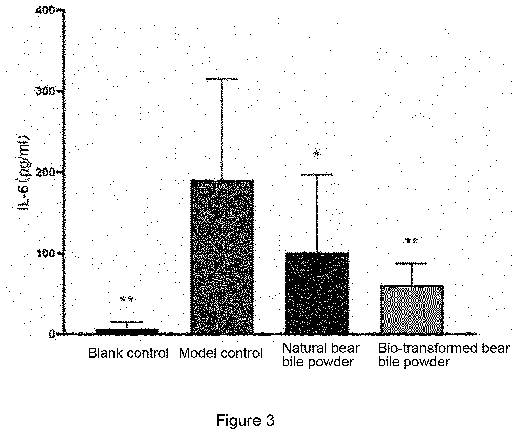

2.1 The effect of bio-transformed bear bile powder on LPS-induced lung inflammation serum and alveolar lavage fluid inflammatory factor IL-6 in mice, as shown in Table 4, Figures 3-4.

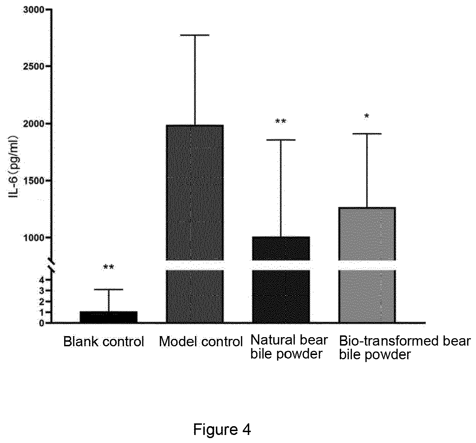

Figure 3 shows the effect of administration of bear bile powder for 7 consecutive days on the inflammatory factors IL-6 in LPS-induced lung inflammation serum in mice, and figure 4 shows the effect of administration of bear bile powder for 7 consecutive days on the inflammatory factors IL-6 in LPS-induced lung inflammation alveolar lavage fluid in mice.

Table 4

| Groups | Dose (mg/kg) | Number of animal | IL-6 concentration (pp/ml) | | | |
|---|---|---|---|---|---|---|
| | | | Day1 | Day 4 | Day 7 | Alveolar lavage fluid |
| Blank control group | - | 12 | 5±6 | 8±8** | 7±8** | 1±2** |
| Model group | - | 10 | 180±92 | 167±92 | 191±118 | 1989±744 |
| Natural bear bile powder | 1000 | 12 | 290±287 | 107±57 | 101±92* | 1008±811** |
| Bio-transformed bear bile powder | 1000 | 11 | 318±211 | 109±54 | 61±25** | 1268±612* |

Note: compared with the model control group, * means p<=0.05, and ** means P<0.01

It can be seen from Table 4 and Figures 3-4 that C57BL/6 mice were exposed to nebulized LPS to induce lung inflammation. Mice in each group were administered intragastrically with the natural bear bile powder 1000 mg/kg and the bio-transformed bear bile powder 1000 mg/kg. The IL-6 levels in the serum of mice were detected on day 1 and day 4, and it was observed that the IL-6 levels detected were not significantly different from those detected in the serum of mice in the model control group. On the 7th day of the administration of the natural bear bile powder 1000 mg/kg and the bio-transformed bear bile powder 1000 mg/kg, it was detected that IL-6 levels in the serum of the mice were significantly reduced when compared with those in the serum of mice in the model control group (P<0.05 or P<0.01). The IL-6 levels in alveolar lavage fluid in mice in the natural bear bile powder 1000 mg/kg group and the bio-transformed bear bile powder 1000 mg/kg group were significantly reduced when compared with those in the alveolar lavage fluid of mice in the model control group (P<0.05 or P<0.01).

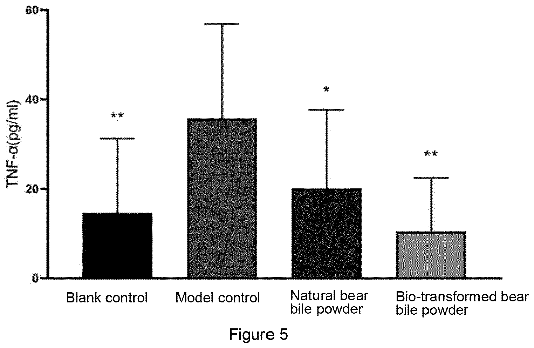

2.2 The effect of bio-transformed bear bile powder on LPS-induced lung inflammation serum and alveolar lavage fluid inflammatory factor TNF-$\alpha$in in mice, as shown in Table 5, Figures 5-6.

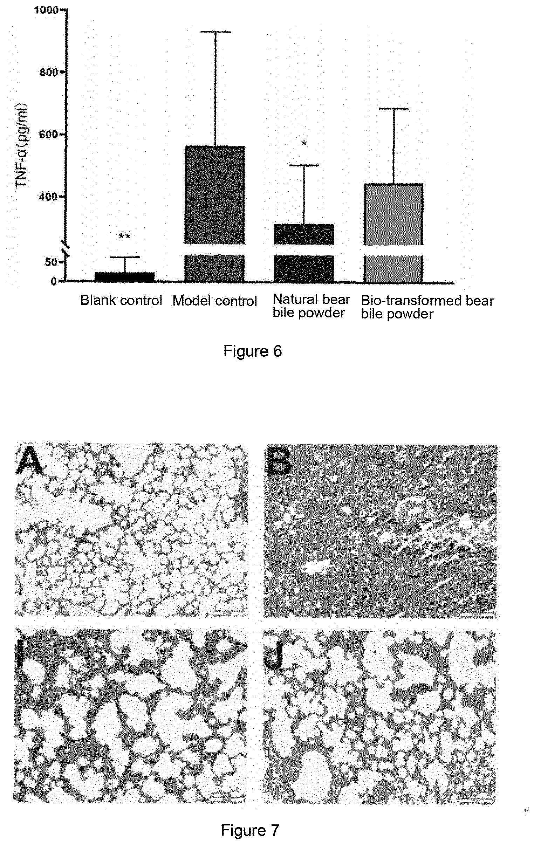

Figure 5 is a graph showing the effect of administration of bear bile powder for 7 consecutive days on the inflammatory factors TNF-$\alpha$ in LPS-induced lung inflammation serum in mice; Figure 6 is a graph showing the effect of administration of bear bile powder for 7 consecutive days on the inflammatory factors TNF-$\alpha$ in LPS-induced lung inflammation alveolar lavage fluid in mice.

Table 5

| Groups | Dose (mg/kg) | Number of animal | TNF-$\alpha$ concentration (pg/ml) | | | |
|---|---|---|---|---|---|---|
| | | | Day 1 | Day 4 | Day 7 | Alveolar lavage fluid |
| Blank control group | - | 12 | 0.1±0.1 | 0.1±0.1 | 14.6±15.9** | 24.2±37.4 |
| Model control group | - | 10 | 7.4±18.2 | 15.1±31.4 | 35.8±20 | 565.4±347.5 |
| Natural bear bile powder | 1000 | 12 | 7.3±10.7 | 14±22.8 | 20.1±16.9* | 316±180.5* |
| Bio-transformed bear bile powder | 1000 | 11 | 7.3±12.4 | 2.8±5.7 | 10.5±11.4** | 448.3±228.2 |

Note: compared with the model control group, * means p<=0.05, and ** means P<0.01

It can be seen from Table 5 and Figures 5-6 that C57BL/6 mice were exposed to nebulized LPS to induce lung inflammation. The level of inflammatory factor TNF-$\alpha$ in the serum and alveolar lavage fluid of mice in each group was detected. It was observed that TNF-$\alpha$ levels detected in the serum of mice on day 1 and day 4 were not significantly different from those detected in the serum of mice in the model control group. On the 7th day of the administration of the

9

natural bear bile powder 1000 mg/kg and the bio-transformed bear bile powder 1000 mg/kg, it was detected that TNF-$\alpha$ levels in the serum of the mice were significantly reduced when compared with those in the serum of mice in the model control group (P<0.05 or P<0.01). The bio-transformed bear bile powder 1000 mg/kg were administered intragastrically. It was detected on the 7th day of the administration that TNF-$\alpha$ concentration in the mice alveolar lavage fluid was reduced compared with that detected in the alveolar lavage fluid of mice in the model control group (P>0.05). The natural bear bile powder 1000 mg/kg was administered intragastrically. It was detected on the 7th day of the administration that TNF-$\alpha$ concentration in the mice alveolar lavage fluid was significantly reduced compared with that detected in the alveolar lavage fluid of mice in the model control group (P<0.05).

Experimental results show that administration of bear bile powder and the bio-transformed bear bile powder to the LPS-induced C57/BL6 mouse models can significantly reduce the levels of inflammatory factors IL-6 and TNF-$\alpha$ in the lung and serum, indicating that both the bear bile powder and the bio-transformed bear bile powder have the anti inflammatory effects. Thus, it proves that the bio-transformed bear bile powder can be used to prepare anti-inflammatory drugs and has optimum pharmacodynamic activity.

2.3 The effect of bio-transformed bear bile powder on the pathological change of lung tissue in mice having LPS-induced lung inflammation

As shown in Figures 7-8, under the 20x objective lens, the alveolar interval of the blank control group was fine, and inflammatory cell infiltration was not so obvious (Figure 7A); while the alveolar septum in the model group was thickened and the alveolar cavity was disappeared. Such changes were similar to fibrosis, in which a large number of inflammatory cells clustered around the bronchi and small blood vessels (Figure 7B).

The following scoring criteria are used to score the lung injury of mice in each group :

no alveolar structural damage in the field of view--0
structural damage is visible in 0-25% field of view--1
structural damage is visible in 25-50% field of view--2
structural damage is visible in 25-50% field of view--3
diffuse injury-4

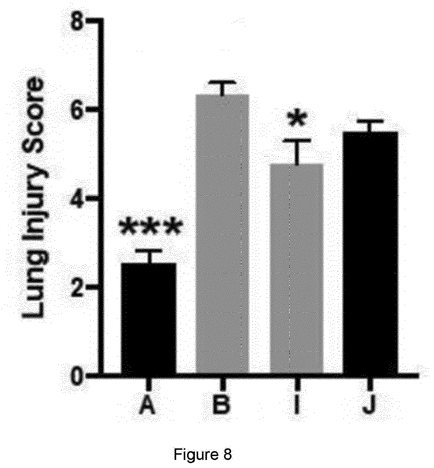

after the bio-transformed bear bile powder and the natural bear bile powder are given to the mice, it is detected that the natural bear bile powder 1000 mg/kg (Figure 8I) and the bio-transformed bear bile powder 1000 mg/kg (Figure 8J) are significantly effective in curbing pathological changes of lung tissue in mice having lung inflammation.

Results show that both the natural bear bile powder and the bio-transformed bear bile powder are effective in improving the LPS-induced lung injury in mice.

The above descriptions are only the preferred embodiments of the invention, not thus limiting the embodiments and scope of the invention. Those skilled in the art should be able to realize that the schemes obtained from the content of specification and drawings of the invention are within the scope of the invention.

**Claims**

1. A use of bio-transformed bear bile powder in preparation of anti-inflammatory drugs.

2. The use of bio-transformed bear bile powder in preparation of anti-inflammatory drugs of claim 1, wherein the anti-inflammatory drugs comprise the bio-transformed bear bile powder and pharmaceutically acceptable excipients.

3. The use of bio-transformed bear bile powder in preparation of anti-inflammatory drugs of claim 1, wherein the dosage form of the anti-inflammatory drugs comprises oral dosage forms or non-oral dosage forms.

4. The use of bio-transformed bear bile powder in preparation of anti-inflammatory drugs of claim 3, wherein the oral dosage forms comprise tablets, powders, granules, capsules, emulsions, syrups, or sprays.

5. The use of bio-transformed bear bile powder in preparation of anti-inflammatory drugs of claim 3, wherein the non-oral dosage forms comprise injections, external preparations.

6. The use of bio-transformed bear bile powder in preparation of anti-inflammatory drugs of claim 1, wherein the bio-transformed bear bile powder is artificial bear bile powder prepared from poultry bile as raw material through biological fermentation.

**7.** The use of bio-transformed bear bile powder in preparation of anti-inflammatory drugs of claim 6, wherein the poultry bile is extracted from one or more selected from the group consisting of chick bile powder, duck bile powder, goose bile powder, cow bile powder, rabbit bile powder, dog bile power, and sheep bile powder.

**8.** A use of bio-transformed bear bile powder in preparation of anti-pneumonia drugs.

**9.** The use of bio-transformed bear bile powder in preparation of anti-pneumonia drugs of claim 8, wherein the anti-pneumonia drugs comprise bio-transformed bear bile powder and pharmaceutically acceptable excipients.

**10.** The use of bio-transformed bear bile powder in preparation of anti-pneumonia drugs of claim 8 wherein the dosage form of the anti-pneumonia drugs comprises oral dosage forms or non-oral dosage forms.

**11.** The use of bio-transformed bear bile powder in preparation of anti-pneumonia drugs of claim 10, wherein the oral dosage forms comprise tablets, powders, granules, capsules, emulsions, syrups, or sprays.

**12.** The use of bio-transformed bear bile powder in preparation of anti-pneumonia drugs of claim 10, wherein the non-oral dosage forms comprise injections, external preparations.

**13.** The use of bio-transformed bear bile powder in preparation of anti-pneumonia drugs of claim 1, wherein the bio-transformed bear bile powder is artificial bear bile powder prepared from poultry bile as raw material through biological fermentation.

**14.** The use of bio-transformed bear bile powder in preparation of anti-pneumonia drugs of claim 13, wherein the poultry bile is extracted from one or more selected from the group consisting of chick bile powder, duck bile powder, goose bile powder, cow bile powder, rabbit bile powder, dog bile powder, and sheep bile powder.

Figure 1

Figure 2

Figure 3

Figure 4

Figure 5

Figure 6

Figure 7

Figure 8

Europäisches
Patentamt

European
Patent Office

Office européen
des brevets

# EUROPEAN SEARCH REPORT

**Application Number**

EP 20 18 5185

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | CN 104 382 941 B (SHANGHAI KAIBAO PHARMACEUTICAL CO LTD) 29 August 2017 (2017-08-29) * the whole document * | 1-14 | INV. A61K31/575 A61K9/14 A61K35/413 A61P9/00 |
| Y | SHA LI ET AL: "Substitutes for Bear Bile for the Treatment of Liver Diseases: Research Progress and Future Perspective", EVIDENCE-BASED COMPLEMENTARY AND ALTERNATIVE MEDICINE, vol. 2016, 1 January 2016 (2016-01-01), pages 1-10, XP055747700, US ISSN: 1741-427X, DOI: 10.1155/2016/4305074 * the whole document * * paragraph [02.2] * * paragraph [0004] * | 1-14 | |
| Y | CN 109 010 373 A (SHANGHAI KAIBAO PHARMACEUTICAL CO LTD) 18 December 2018 (2018-12-18) * the whole document * | 1-14 | TECHNICAL FIELDS SEARCHED (IPC) A61K A61P |
| Y | JOO SEONG-SOO ET AL: "Anti-inflammatory Effect of Bear's Gall in Rat Microglia", THE KOREAN ASSOCIATION OF ORIENTAL MEDICAL PHYSIOLOGY, KOREAN INTELLECTUAL PROPERTY OFFICE, vol. 19, no. 1, 25 February 2005 (2005-02-25), pages 204-211, XP053003204, ISSN: 1226-0436 * the whole document * | 1-14 | |

The present search report has been drawn up for all claims

| Place of search | Date of completion of the search | Examiner |
|---|---|---|
| Munich | 6 November 2020 | Collura, Alessandra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 5185

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2020

| Patent document cited in search report | | Publication date | Patent family member(s) | Publication date |
|---|---|---|---|---|
| CN 104382941 | B | 29-08-2017 | NONE | |
| CN 109010373 | A | 18-12-2018 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201410588581

- CN 104382941 B